Europäisches Patentamt

European Patent Office

Office européen des brevets

(19)

(11) Publication number: **0 092 875**
**A1**

## (12) EUROPEAN PATENT APPLICATION

(21) Application number: **83200572.2**

(22) Date of filing: **20.04.83**

(51) Int. Cl.³: **C 07 H 15/04**

(30) Priority: **26.04.82 US 371698**
**25.03.83 US 476750**

(43) Date of publication of application:
**02.11.83 Bulletin 83/44**

(84) Designated Contracting States:
**BE DE FR GB IT NL SE**

(71) Applicant: **THE PROCTER & GAMBLE COMPANY**
**301 East Sixth Street**
**Cincinnati Ohio 45202(US)**

(72) Inventor: **Mao, Mark Hsiang K.**
**4114 Fox Hollow Drive**
**Cincinnati, Ohio 45241(US)**

(74) Representative: **Ernst, Hubert et al,**
**PROCTER & GAMBLE EUROPEAN TECHNICAL CENTER**
**Temselaan 100**
**B-1820 Strombeek-Bever(BE)**

(54) Process of preparing alkylpolysaccharides.

(57) An improved process for making long chain alkyl polysaccharides by reacting a long chain alcohol with a short chain alkyl saccharide in the presence of an acid catalyst in which the temperature and time of reaction are carefully controlled to avoid discoloration and excessive polymerization.

EP 0 092 875 A1

## 0092875

### PROCESS OF PREPARING ALKYLPOLYSACCHARIDES
Mark H. K. Mao

### Technical Field and Background Art

This invention relates to an improved process for making alkylpolysaccharides in which the alkyl group contains from about 12 to about 18 carbon atoms and the polysaccharide chain contains from about $1\frac{1}{2}$ to about 20 saccharide units on the average. The improved process gives a good polysaccharide chain length distribution and a good color. The process can be used to prepare highly efficient alkylpolysaccharides useful as detergent surfactants and foam builders for anionic detergent surfactants.

### Summary of the Invention

The present invention relates to a process for preparing a long chain alkyl polysaccharide comprising the essential step of reacting a short chain alkylsaccharide of a reducing saccharide containing from about five to about six carbon atoms in which the alkyl contains from one to about five carbon atoms with a long chain fatty alcohol containing from about 12 to about 18 carbon atoms at a reaction temperature of from about 90°C to about 120°C in the presence of an acid catalyst, the resulting short chain alcohol being removed, preferably under a vacuum, and preferably as rapidly as possible, with the catalyst being destroyed by adding an alkaline material after at least about 90% of the short chain alkyl saccharide has been destroyed, the extent of destruction can be determined by measuring the resulting short chain alcohol which has been removed, or, preferably, by measuring the percent of water in the distillate, and before the average polysaccharide chain length exceeds about 20, preferably before the length exceeds about 4, most preferably before the length exceeds about 3.

In a second highly preferred step the unreacted long chain fatty alcohol in the product from the above step is removed by applying a vacuum and heat to a thin film of the product of the first step whereby the unreacted fatty alcohol is reduced to a level of less than about 2%, preferably less than about $\frac{1}{2}$%.

The combination of the above two steps can be used to provide a superior alkylpolysaccharide for use as a detergent surfactant in which the alkyl group contains from about 12 to about 18, preferably from about 12 to about 14 carbon atoms, the average polysaccharide chain length is from about 1½ to about 3, preferably from about 1.6 to about 2-3/4 saccharide units, the level of short chain alkylsaccharide and polysaccharide is less than about 10%, the amount of alkylpolysaccharide in which the saccharide chain length is 6 or greater is less than about 10%, preferably less than about 5%, the alkylmonosaccharide content is less than about 60%, preferably less than about 50%, and the unreacted fatty alcohol content is less than about 2%, preferably less than about ½%.

## Detailed Description of the Invention

The fatty alcohols useful herein may be primary or secondary alcohols having straight or branched chains which can be either saturated or unsaturated, and may contain ether linkages. Preferably, the alcohols are primary saturated alcohols. Examples include dodecyl, tridecyl, tetradecyl, pentadecyl, hexadecyl, and octadecyl alcohols, and mixtures thereof. The preferred fatty alcohols are those containing from about 12 to about 14 carbon atoms.

The short chain alkylmonosaccharides, are e.g., the methyl, ethyl, propyl, butyl, and pentyl, preferably propyl or butyl, and most preferably butyl, fructosides, glucosides, mannosides, galactosides, talosides, allosides, altrosides, idosides, arabinosides, xylosides, lyxosides, ribosides, and mixtures thereof. The preferred alkylmonosaccharides are glucosides and fructosides due to their availability and low cost, and the most preferred alkylmonosaccharide is derived from glucose. These compounds can be prepared separately, in a preliminary step, or as part of the first step since the corresponding short chain alcohols react with the corresponding reducing saccharides containing from five to six carbon atoms much faster than the long chain fatty alcohols react. Saccharides containing six carbon atoms are preferred.

The molar ratio of the long chain fatty alcohol to the short chain alkylmonosaccharide is between about 1:4 and about 4:1, preferably between about 1:2 and about 2:1, most preferably between about 1:1 and about 1.2:1. The level of long chain fatty alcohol is preferably kept as low as possible to facilitate the removal of unreacted fatty alcohol from the desired alkyl polysaccharide. An auxiliary solvent can be used to maintain fluidity. The lower levels of fatty alcohol also maximize the formation of the desired long chain alkylpolysaccharides especially at levels above about 60%. Preferably the level of long chain alkylmonosaccharide in the finished product is less than about 60%, most preferably less than about 50%.

The reaction is carried out at a temperature of from about 90°C to about 120°C, preferably above about 100°C. Above about 120°C there is excessive formation of colored materials and excessively fast saccharide chain growth. However, the reaction temperature should be as high as possible to minimize the time of the reaction.

If an auxiliary solvent is used, it should have a boiling point that will permit its easy removal for recycling. It should also be compatible with the short chain alcohol, the long chain alcohol, the saccharide and the alkyl saccharides and should not be reactive. Suitable auxiliary solvents include: toluene, $C_{8-12}$ hydrocarbons, etc.

It is desirable that the resulting product contain a minimum of the short chain alkylsaccharides, which do not provide any substantial detergency benefits. However, care must be taken in removing the last amount of the short chain alcohol since this normally requires more stringent conditions and one wants to avoid removal of the long chain alcohol. Furthermore, if the reaction proceeds for too long a time, the average polysaccharide chain length becomes too long. Increasing the reaction time can be used to achieve longer polysaccharide chains since free saccharide reacts with the end of the saccharide chain preferentially as compared to the fatty alcohol. Accordingly, it is desirable to remove the short chain fatty alcohol rapidly and kill the catalyst by adding an alkaline material as soon as the desired product is achieved.

Known analytical techniques can be used to determine the structures of the alkylpolysaccharide surfactants herein; for example, to determine the saccharide chain length, the amount of butyl glucoside, the free fatty alcohol content, and the level of unreacted polysaccharide. More specifically, gas or liquid chromatography can be used to determine the unreacted alcohol content and the unreacted polysaccharide content respectively. Proton nmr can be used to determine the average saccharide chain length. The point of attachment of the hydrophilic portion of the molecule to the hydrophobic portion of the molecule can be determined by $^{13}$C nmr.

The alkylpolysaccharide surfactants are complex mixtures. Their components vary depending upon the nature of the starting materials and the reaction by which they are preapred. Analytical standards which are useful in calibrating instruments for analyzing the components of a particular alkylpolysaccharide surfactant can be obtained from Calbiochem Behring Co. LaJolla, California. These standards include those for octylglucoside (Calbiochem #494559), decylglucoside (Calbiochem #252715), and dodecylmaltoside (Calbiochem #3243555).

The combination of vacuum and temperature should not remove the long chain alcohol. Preferably the reaction takes place in a thin film, preferably at high Reynolds numbers (>720,000), as set forth hereinafter, to permit rapid removal of the short chain alcohol which results, and preferably the reaction takes place under a vacuum to assist in the rapid removal of the resulting short chain alcohol. Thin films can be achieved using, preferably, a wiped film evaporator or a drum evaporator, or mills in which two cylinders combine to form a thin film, etc. In a mill it is desirable that one of the cylinders rotate faster than the other to impart a shearing and mixing action. The reaction mix is conveniently removed from a drum or cylinder by a doctor blade.

The length of the saccharide chain is primarily controlled by adjusting the ratio of the saccharide monomer to the fatty alcohol. For any given ratio there is a desired end point at which time the desired reaction is complete and beyond that point one effects undesired dehydration of the saccharide moieties.

The synthesis process can be monitored by following the percent of water contained in the distillate. (The distillate contains mainly lower fatty alcohol and long chain fatty alcohols.) The water level can be analyzed by collecting the distillate in fractions and titrating it with Karl Fischer reagent. The optimum end point is at the lowest water level in the distillate, preferably <0.1%. Further reaction increases the glycoside chain length rapidly and can make the product unsuitable for, e.g., detergent applications.

An in-line moisture monitor is ideal to control the process.

In one reaction in which the initial molar ratio of butyl glycoside to $C_{12-13}$ fatty alcohol was about 3, the temperature was about 118°C and the pressure varied from about one cm. of Hg to one atomosphere, the percent water in the distillate varied with the amount of distillate collected as follows:

| Distillate (ml) | 50 | 140 | 270 | 320 | 360 | 375 | 380 | 396 |
|---|---|---|---|---|---|---|---|---|
| % $H_2O$ | 12.4 | 4.1 | 0.9 | 0.2 | 0.08 | 0.25 | 0.3 | 0.15 |

The desired end point occurs when the percent of water in the distillate is less than about 0.2, preferably less than about 0.1, most preferably less than about 0.08.

The acid catalysts can be any of the conventional acids such as sulfuric acid, hydrochloric acid, phosphoric acid, phosphorus acid, toluene sulfonic acid, etc., and other Lewis acid catalysts, etc. The amount of acid catalyst used is between about 0.0001 mole per mole of saccharide monomer and about 0.02 mole per mole of saccharide monomer, preferably between about 0.005 and about 0.01, most preferably between about 0.001 and about 0.006. The amount of catalyst used can control the speed of reaction. When larger amounts of catalysts are used the neutralized catalyst should be one that is compatible with the end use of the surfactant. Sulfuric acid is the preferred catalyst.

## Removal of the Fatty Alcohol

The fatty alcohol can be removed from the product of the first step by distillation or by a solvent extraction technique. The fatty alcohol and any auxiliary solvent are removed after the catalyst has been destroyed by neutralization. The preferred method of fatty alcohol removal is to form a thin film of the reaction product containing the neutralized catalyst and apply heat and a vacuum. A wiped film evaporator is a particularly preferred piece of equipment for removing the fatty alcohol. Although the prior art represented by German OLS. 3,001,064 has described the problem associated with removing fatty alcohol from an alkyl polysaccharide without causing an increase in the color, it has now been found that the use of a thin film evaporator and a vacuum permits the removal of the fatty alcohols and/or solvent of this invention to a level below about 2% and even below about 1/2% without any appreciable change in the color of the product. This discovery is especially important when one is preparing alkylpolysaccharides from alcohols containing more than 10 carbon atoms where the boiling point of the fatty alcohol, even under a high vacuum, is very high and prolonged exposure to high temperature leads to decomposition of the polysaccharide. With alkyl polysaccharides in which the alkyl group contains more than 10 carbon atoms, it is preferred to have a small amount of short chain alkyl ($C_{1-5}$) saccharide present to maintain the fluidity thus allowing lower temperatures to be used.

In the removal process of this invention the product is formed into a thin film at a temperature of from about 120°C. to about 200°C., preferably from about 140°C. to about 180°C., most preferably from about 160°C. to about 170°C. and under a vacuum of from about 0.1mmHg to about 20mmHg, preferably from about 0.1mmHg to about 5mmHg, most preferably from about 0.1mmHg to about 3mmHg. The thin film during the reaction and the removal steps is preferably formed in a thin film evaporator which gives a film with a thickness of from about 1mm to about 10mm and a Reynolds number of at least about 20,000, preferably at least about 50,000, and more preferably about 100,000. The film in such evaporator is preferably less than about 5mm at its thinnest and more preferably less than about 5mm in the wave.

### Preferred Product

The process defined hereinbefore can be used to prepare a preferred alkylpolysaccharide detergent surfactant having superior properties with respect to detergency and suds boosting for other detergent surfactants. This preferred alkylpolysaccharide has the formula $RO(Z)_x$ is which R is an alkyl group containing from about 10 to about 18 carbon atoms; Z represents a 5 or 6, preferably 6, carbon member reducing saccharide moiety; x averages from about 1.6 to about 2-3/4; the amount of alkylpolysaccharide in which x is greater than 6 is less than about 10%, preferably less than about 5%; the amount of the alkylpolysaccharide in which x is 1 is less than about 60%, preferably less than about 50%, and the defined material is associated with no more than about 10% alkylsaccharides and polysaccharides wherein the alkyl group contains less than about 8 carbon atoms and with no more than about 2% alcohol containing an R group.

Preferably in the above compound R is a straight chain saturated alkyl group and preferably the R groups contain from about 12 to about 14 carbon atoms. An even more preferred average of x is from about 1.7 to about 2.5. The preferred Z group is a glucoside group.

The above preferred alkylpolysaccharide preferably constitutes at least about 90% of the alkylpolysaccharide material present. It is difficult to achieve an alkylpolysaccharide having the appropriate alkyl chain length and average x without either exceeding the desired amount of fatty alcohol or monosaccharide on the one hand, or providing excess material in which x exceeds 6 on the other hand. It is also difficult to maintain a good color in alkyl polysaccharide detergent surfactants to permit their incorporation into detergent compositions, and to minimize the amount of short chain alkylpolysaccharide present.

The process described hereinbefore achieves the desired product by minimizing the temperature to which the material is exposed, especially while an acid catalyst is present; maximizing the speed at which the short chain alcohol is removed; killing the catalyst as soon as the desired end point has been reached; and then removing the fatty alcohol, preferably by a process which minimizes the time and the temperature to which the desired product is exposed.

Surprisingly, it has been discovered that the increase in the alkyl chain length from 10 to 12 results in a very large decrease in the reactivity of the fatty alcohol. It also increases considerably the difficulty involved in removing the fatty alcohol without exceeding the decomposition temperature of the polysaccharide chain. The combination process disclosed herein achieves the desired product.

The importance of the limits on the preferred alkylpolysaccharide detergent surfactant are documented hereinafter in the examples. In order to obtain maximum performance, x needs to be as low as possible while maintaining water solubility. The desired products have an HLB of from about 7 to about 30, preferably from about 10 to about 20, and a critical micelle concentration of from about 10 ppm to about 1000 ppm, preferably from about 20 ppm to about 500 ppm.

### EXAMPLE I

50ml of n-butanol, 10g anhydrous glucose, 20ml n-dodecanol and 0.0534g of p-toluene sulfonic acid were added to a 100ml 3-neck flask with stirring. The reaction mixture was refluxed at 115-117°C for 2 hours. The n-butanol was then removed as fast as possible with the help of partial vacuum while keeping the temperature at 100°C. to 120°C. The reaction was kept at 120°C, 5cm Hg vacuum for 40 minutes. 0.027g of $Na_2CO_3$ was used to neutralize the reaction mixture. The unreacted n-dodecanol was then distilled off using a Wiped Film Evaporator by Pope at a temperature of 165°C and a vacuum of 2 mm Hg. The final sample is a crispy solid with the following analyses: (In the following, e.g.,

dodecanol polyglycoside with n glucose moieties is abbreviated $C_{12}G_n$). Average glucose number per alkyl chain: 2.0, weight percent of n-butyl oligoglycoside: <5.9%, $C_{12}, G_1$ : ~40%, $C_{12}G_2$ : 21%, $C_{12} G_3$ 13%, $C_{12} G_4$ 9.8%, $C_{12} G_5$ 8%, >$C_{12} G_6$, <10%, $C_{12}$-OH : 0.46%.

## EXAMPLE II

500ml n-butanol, 48g anhydrous glucose, 200 ml Neodol 23 and 0.0534g of p-toluene sulfonic acid were reacted. The reaction was carried out in a 1000ml flask with vigorous stirring. After n-butanol removal, reaction condition was set at 118°C, 2cmHg vacuum until 103% of the added n-butanol volume was collected. (Water) After neutralizing in the usual manner, the sample was dried, as in Example I, through the wiped film evaporator. The product was analyzed to be: average glycoside number per alkyl chain: 2.0, weight percent of n-butyl oligoglycoside <1%, $C_{12,13} G_1$ ~ 49%, $C_{12,13} G_2$ : 19%, $C_{12,13} G_3$ : 11.5%, $C_{12,13} G_4$ : 8.4%, $C_{12,13} G_5$ : 4.44%, $C_{12,13} G_6$ : 3.85%.

## EXAMPLE III

500 ml n-butanol, 192g anhydrous dextrose, 200 ml Neodol 23 and 0.0534g p-toluene sulfonic acid. The reaction was carried out in the same manner as Example II. The final product was analyzed to be: average glucose number per alkyl chain: <2.5, $C_{12,13} G_1$ : 35%, $C_{12,13} G_2$ : 20%., $C_{12,13} G_3$ : 15%, $C_{12,13} G_4$ : 12.6%, $C_{12,13} G_5$ : 9.8%, $C_{12,13}G_6$ : 7.6%.

## COMPARATIVE EXAMPLE IV

480g anhydrous dextrose, 6.0g p-toluene sulfonic acid, 960g Neodol-23 ($C_{12-13}$ fatty alcohol) and 3600ml n-butanol were refluxed at 117°C for 1 hour. The n-butanol was distilled off under atmospheric pressure for 45 minutes at 120°C. The pressure was reduced gradually to 50mm Hg and the temperature was maintained at 130°C for 1.5 hours. The reaction mixture was neutralized with 3g sodium carbonate in 20ml $H_2O$. The excess alcohol was washed away with acetone. The product had the following analyses: 27% butyl polyglycosides, 10.2 glucose units/per molecule. This indicated the temperature was too high and too much catalyst was used. This favored the polysaccharide chain length polymerization without removing the butyl polyglycosides.

## EXAMPLE V

96g anhydrous dextrose, 200ml Neodol-23 ($C_{12-13}$ fatty alcohol), 0.4g p-toluene sulfonic acid and 500ml n-butanol were refluxed for 2 hours. 95% of the n-butanol was then removed by distillation at 115°C $\pm$ 5°C under vacuum. The resulting mixture was then passed through a Pope 2-inch Wiped Film Evaporator twice, operated at 120°C, and 2cm Hg pressure. The reaction mixture before the removal of excess fatty alcohol was the following composition: alkyl polyglycoside: 51%, average glucose per molecule: 2.0, butyl polyglycosides: <5%.

## EXAMPLE VI

400g anhydrous dextrose, 800ml Neodol-91 ($C_{9-11}$ fatty alcohol), 1.6g p-toluene sulfonic acid and 2000ml n-butanol were refluxed for 2 hours. 95% of the butanol was then removed under the same conditions as in Example V. The reaction mixture was passed through a Pope Wiped Film Evaporator twice, the resulting mixture contains: alkyl polyglycoside 48%, average glucose per molecule 1.6, butyl polyglycoside <3%.

### Wiped Film Evaporator Examples

## EXAMPLE VII

960g anhydrous dextrose, 2000ml Neodol-23, 5000ml n-butanol and 1.2g concentrated sulfuric acid were refluxed at 117°C for 2 hours. n-butanol was then removed at 117°C, 2cm Hg pressure. The reaction mixture was neutralized with $Na_2CO_3$ solution. The excess Neodol-23 was then removed by a 2 inch Pope Wiped Film Evaporator operated at 160-170°C, 2mm Hg pressure. The solution residence time in the hot zone was approximately 1 minute. The resulting product had a light brown color. Melting point about 130°C. It was cooled to room temperature and was easily ground into a yellowish powder. Analyses showed a content of 0.45% Neodol-23. It dissolved readily in water and gave a yellowish clear solution.

## EXAMPLE VIII

100g anhydrous dextrose, 200ml Neodol-91, 500ml n-butanol and 0.1g concentrated sulfuric acid were refluxed at 117°C for 2 hours. n-butanol was then removed at 117°C and 2cm Hg pressure. After neutralization by $Na_2CO_3$, the reaction mixture was passed through a 2 inch Pope Wiped Film Evaporator. The operating conditions were 155-165°C and 2mm Hg pressure. A product containing 0.2% Neodol-91 was obtained.

## EXAMPLE IX

The same reaction as in Example VIII was carried out except 400ml Neodol-23 was used in place of Neodol-91. The reaction mixture was passed twice through the Pope Wiped Film Evaporator at 165-170°C and 2mm Hg pressure. The product contained 0.4% Neodol-23.

## EXAMPLE X

420 ml of 1-butanol, 0.7 g p-toluene sulfonic acid, 57g methyl α-D-glucopyranoside and 25g methyl β-D-glucopyranoside were added to a one liter, 3 neck flask. The above mixture was refluxed at 116-118°C. After 2-3 hours of refluxing, the boiling point of the mixture dropped to 109°C. 230ml of a mixture of dodecanol and tridecanol was added. The mixture was allowed to boil at 118°C and the distillate collected. Methanol was collected first, followed by butanol. Vacuum was slowly increased to give a steady rate of distillation without letting the temperature rise above 120°C. The level of moisture contained in the distillate was measured by Karl Fischer titration. The reaction was stopped when the moisture level dropped to below 0.1% in the distillate. 0.27g $Na_2CO_3$ dissolved in 3ml water were added to neutralize the catalyst. The reaction mixture was then purified by removing excess dodecanol and tridecanol using a Kugelrohr distillation apparatus. (Aldrich Cat. No. 210,046-3). The resulting cake was ground to give a biege colored powder. Analysis gave 2.0 average glycoside units per molecule, 8.0% butyl oligoglycosides and no methyl glucosides.

## EXAMPLE XI

500ml 1-butanol, 100g galactose, 0.5g p-toluene sulfonic acid were added to a one liter 3-necked flask. The mixture was refluxed at 116-118°C until clear. About 280ml butanol was then removed through distillation. 400ml of a mixture of dodecanol and tridecanol was added and the mixture was distilled at 120°C with partial vacuum. The reaction was stopped when the amount of water contained in the distillate dropped below 0.1%. Sodium carbonate and citric acid buffer were added to neutralize the catalyst. The mixture was distilled to remove excess fatty alcohol. The resulting product was found to be suitable for detergent applications.

CLAIMS

1. The process for preparing alkyl polysaccharides in which the alkyl group contains from about 12 to about 18 carbon atoms comprising the step of reacting a short chain alkyl monosaccharide of a reducing saccharide containing from about five to about six carbon atoms, in which the alkyl group contains from 1 to about 5 carbon atoms with a fatty alcohol containing from about 12 to about 18 carbon atoms at a temperature of from about 90°C. to about 120°C. in the presence of an acid catalyst while removing the resulting alcohol containing from 1 to about 5 carbon atoms and destroying the acid catalyst after at least 90% of the short chain alkyl monosaccharide has been destroyed and before the average polysaccharide chain length exceeds about 20.

2. The process of Claim 1 wherein the temperature is from about 100°C. to about 120°C. and the catalyst is present at a level of from about 0.0001 mole to about 0.02 mole per mole of saccharide monomer.

3. The process of Claim 2 wherein the catalyst is destroyed after at least 95% of the short chain alkyl monosaccharide has been destroyed and before the average polysaccharide chain length exceeds about 4.

4. The process of Claim 1 wherein the reaction takes place in a thin film.

5. The process of Claim 1 wherein the destroying of the acid catalyst takes place after the percent of water in the distillate is below about 0.2%.

6. The process of Claim 5 wherein the catalyst is destroyed after the percent of water in the distillate is below about 0.1%.

7. The process of Claim 6 wherein the catalyst is destroyed after the percent of water in the distillate is below about 0.08%.

European Patent
Office

**EUROPEAN SEARCH REPORT**

0092875
Application number

EP 83 20 0572

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | DE-A-1 905 523 (ATLAS) <br> * Pages 1-28 * | 1-7 | C 07 H 15/04 |
| X | DE-A-2 036 472 (ATLAS) <br> * Pages 1-20 * | 1-7 | |
| | | | **TECHNICAL FIELDS SEARCHED (Int. Cl. 3)** |
| | | | C 07 H 15/00 |

The present search report has been drawn up for all claims

| Place of search THE HAGUE | Date of completion of the search 12-07-1983 | Examiner VERHULST W. |
|---|---|---|